# EUROPÄISCHE PATENTSCHRIFT

(11) **EP 3 542 367 B1**
(45) Veröffentlichungstag und Bekanntmachung des Hinweises auf die Patenterteilung: **11.02.2026**
(21) Anmeldenummer: 17811203.3
(22) Anmeldetag: 15.11.2017
(51) Int. Cl.: G16H 40/67, G16H 40/20

(54) **KONZERTIERTE ALARMBEHANDLUNG FUER EINEN VERBUND VON DIALYSEGERAETEN**
CONCERTED ALARM PROCESSING FOR A GROUP OF DIALYSIS DEVICES
TRAITEMENT CONCERTÉ DES ALARMES D'UN ENSEMBLE D'APPAREILS DE DIALYSE

(30) Priorität: 16.11.2016 DE 102016121980
(43) Veröffentlichungstag der Anmeldung: 25.09.2019
(73) Patentinhaber: Fresenius Medical Care Deutschland GmbH, 61352 Bad Homburg (DE)
(72) Erfinder: WEGNER, Stefan, 97440 Werneck (DE)
(74) Vertreter: Schwarz, Claudia
(86) Internationale Anmeldenummer: PCT/EP2017/079257
(87) Internationale Veröffentlichungsnummer: WO 2018/091492

(56) Entgegenhaltungen:
- US-A1- 2011 107 251

## Beschreibung

Die vorliegende Erfindung betrifft die technische Bearbeitung von Alarmmeldungen und Warnungen von Dialysegeräten und bezieht sich insbesondere auf ein vernetztes Statusmeldungssystem, einen Steueragenten und auf ein Verfahren zum Steuern eines Verbundes von Dialysegeräten bei der Behandlung von Meldungen, insbesondere Alarmmeldungen.

Dialysegeräte werden in Dialysekliniken eingesetzt, in denen in der Regel mehrere Patienten gleichzeitig behandelt werden. Das Fachpersonal ist dabei mit einer Vielzahl von Arbeitsschritten befasst und ist verantwortlich für den fehlerfreien Betrieb eines Verbundes von Geräten. Bei bestimmten Betriebsbedingungen, in Ausnahmesituationen oder im Fehlerfall werden vom Dialysegerät automatisch Meldungen erstellt, die die jeweilige Ausnahmesituation oder den Fehler repräsentieren. Diese Meldungen können in einem optischen und/oder akustischen Format ausgegeben werden. Die Meldungen können z.B. einen Fehlerzustand am Gerät signalisieren. Die Meldungen sind vom Anwender zu quittieren. Handelt es sich um eine Fehlermeldung, muss in der Regel eine Aktion am Gerät ausgeführt werden, um das Gerät möglichst rasch wieder in einen betriebsfähigen Zustand zu überführen. Betrifft die Meldung beispielsweise den Hinweis, dass ein Blutverlust im extrakorporalen Blutkreislauf detektiert wurde, so hat diese Alarmmeldung eine hohe Priorität, um umgehend die Ursache für diesen Fehler zu finden und die Sicherheit für den Patienten weiterhin gewährleisten zu können, damit das Gerät wieder in einem fehlerfreien Betriebsmodus überführt werden kann. Der Anwender wird die Meldung somit zunächst bestätigen bzw. quittieren, um daraufhin geeignete Maßnahmen zu ergreifen. Kann der Fehler erfolgreich behoben werden, so wird das vom Gerät erkannt, so dass die Meldung nicht mehr erscheint.

In der Regel sind die heutigen, modernen medizintechnischen Geräte, wie beispielsweise das Hämodialysesystem 5008 von Fresenius Medical Care, mit einer (hier: optischen) Anzeigevorrichtung zur Ausgabe von Meldungen in Bezug auf den Status des medizintechnischen Gerätes ausgestattet. Die Anzeigevorrichtung kann in der Art einer Ampel betrieben werden und signalisiert beispielsweise mit einem grünen Lichtsignal einen fehlerfreien Betrieb und mit einem roten Lichtsignal (z.B. im Blinkmodus) einen Alarm. Durch diese Signalisierung wird es möglich, dass das Fachpersonal bereits von einer entfernten Position erkennen kann, in welchem Zustand sich das Gerät befindet und wie dringend eine Meldung zu behandeln ist.

In der klinischen Praxis ist das fehlerfreie Funktionieren des medizintechnischen Gerätes unabdingbar. Deshalb ist bei den bisherigen Systemen jedes medizintechnische Gerät (z.B. Dialysegerät) mit einer solchen Anzeigevorrichtung ausgebildet, die lokal vom Gerät angesteuert wird. Wie vorstehend erwähnt, werden die Dialysegeräte in der Praxis in der Regel in einer Dialyseabteilung eingesetzt und somit in einem Verbund von Geräten. Aufgrund der lokalen Ansteuerung der Anzeigevorrichtung ist es bei den bisherigen Geräten in nachteiliger Weise nicht möglich, von der Ferne zu unterscheiden, welche Meldung priorisiert zu behandeln ist, da keine Differenzierungsmöglichkeit zwischen den unterschiedlichen Meldungen bereitgestellt wird.

Ausgehend von den bekannten Systemen als Stand der Technik hat sich die vorliegende Erfindung deshalb zur Aufgabe gestellt, die Behandlung von Gerätemeldungen zu verbessern und insbesondere eine Differenzierungsmöglichkeit zwischen den Statusmeldungen der einzelnen Geräte des Geräteverbundes bereitzustellen. Weiterhin soll eine Priorisierung von allen Statusmeldungen für die Geräte des Verbundes möglich sein, um den Zeitraum zur Herstellung eines fehlerfreien Betriebszustandes (durch Ausführen von erforderlichen Maßnahmen) zu verkürzen. Ferner soll der Gerätebetrieb mit Gerätebedienung im Rahmen von Gerätemeldungen verbessert werden, was wiederum die Qualität des Geräteeinsatzes erhöht.

US 2011/0107251 A1 beschreibt eine Benutzerschnittstelle zum Überwachen des Status für mehrere medizinische Maschinen mit einem Display zur Anzeige der Daten. Die Daten werden von einer Verarbeitungseinheit bereitgestellt. Die Verarbeitungseinheit ermittelt, ob sich die jeweilige medizinische Maschine in einem korrekt funktionierenden Zustand, einem Warnzustand oder in einem kritischen Zustand befindet. Die unterschiedlichen Zustände werden mit unterschiedlichen Farben dargestellt. Dabei werden die Maschinen aber jeweils für sich und separat bezüglich ihrer Warnmeldungen behandelt. Es erfolgt aber keine konzertierte Berechnung von priorisierten Warnmeldungen für den Verbund von Maschinen.

Diese Aufgabe wird erfindungsgemäß durch ein vernetztes Statusmeldungssystem, einen Steueragenten und ein Verfahren gemäß den beiliegenden, einander nebengeordneten Patentansprüchen gelöst.

Im Folgenden wird die Erfindung anhand der vorrichtungsgemäßen Aufgabenlösung, und somit u.a. anhand des vernetzten Statusmeldungssystems beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die anderen gegenständlichen Ansprüche (die beispielsweise auf einen Steueragenten gerichtet sind) und die Verfahrensansprüche mit den Merkmalen weitergebildet sein, die in Zusammenhang mit dem System beschrieben oder beansprucht sind. Die entsprechenden funktionalen Merkmale des Verfahrens werden dabei durch entsprechende gegenständliche Module, insbesondere durch elektronische Hardware-Module oder Mikroprozessor-Module, des Systems bzw. der Vorrichtung ausgebildet und umgekehrt.

Gemäß einem ersten Aspekt betrifft die Erfindung ein vernetztes Statusmeldungssystem für einen Verbund von medizintechnischen Geräten. Bei den medizintechnischen Geräten kann es sich insbesondere um Dialysegeräte handeln. Das Statusmeldungssystem umfasst die Merkmale gemäß Patentanspruch 5.

Im Folgenden werden die in dieser Anmeldung verwendeten Begrifflichkeiten definiert.

Die erfassten lokalen Gerätestatusmeldungen betreffen einen Zustand des medizintechnischen Gerätes oder der an das Gerät angeschlossenen technischen Vorrichtungen (Pumpen, Schlauchsysteme etc.) und können insbesondere Fehler- oder Alarmmeldungen sein. Die jeweilige lokale Gerätestatusmeldung kann auch zumindest ein Sensorsignal und/oder andere technische Betriebsparameter des medizintechnischen Gerätes umfassen, aus denen der Zustand des Gerätes mittelbar errechnet werden kann.

Die lokale Signalvorrichtung ist auf jedem medizintechnischen Gerät implementiert und kann eine optische und/oder akustische Anzeigevorrichtung, z.B. in Form einer Ampel zur Anzeige von abgestuft priorisierten Warnmeldungen, umfassen (grün leuchtend für störungsfreien Betrieb, gelb blinkend bei einer Warnung und rot leuchtend oder blinkend für einen Alarm). Im Stand der Technik wird die lokale Signalvorrichtung lediglich lokal auf Basis von lokal erfassten Signalen des jeweiligen medizintechnischen Gerätes angesteuert. Gemäß der Erfindung wird die lokale Signalvorrichtung über den Steueragenten für alle Geräte des Verbundes auf konzertierte Weise (z.B. zentral) angesteuert. Damit werden alle lokalen Signalvorrichtungen auf konzertierte Weise angesteuert. Bei der Ansteuerung wird somit der Zustand der anderen medizintechnischen Geräte berücksichtigt. Die lokale Signalvorrichtung kann einen Prozessor mit einer Auswerteschaltung umfassen. Die lokale Signalvorrichtung ist darüber hinaus mit einer Eingangs- und einer Ausgangs-Schnittstelle zu dem Steueragenten ausgebildet. Die lokale Signalvorrichtung dient zur Ausgabe einer konzertierten Warnmeldung. "Konzertiert" bezieht sich in diesem Kontext darauf, dass die Warnmeldung relativ in Bezug zum Gerätezustand aller anderen Geräte im selben Zeitraum berechnet worden ist. Dies kann an zentraler Stelle erfolgen. Die konzertierte Berechnung kann aber auch lokal auf einem dedizierten Gerät, aber dennoch zentralisiert und für alle Geräte des Verbundes gemeinsam ausgeführt werden.

In einer vorteilhaften Weiterbildung der Erfindung kann die lokale Signalvorrichtung eine Erfassungseinheit umfassen, die zur Erfassung der jeweiligen lokalen Gerätestatusmeldung des Gerätes oder zur Erfassung eines Gerätezustandes bestimmt ist. Die Erfassungseinheit kann in vordefinierten Zeitintervallen oder nach Eintritt von vordefinierbare Ereignissen (z.B. bei Zustandsänderung eines Gerätes des Verbundes) aktiviert werden. Eine Ausgabeeinheit der lokalen Signalvorrichtung dient dann zur Ausgabe der (z.B. zentral) berechneten priorisierten Warnmeldung (und nicht der lokal auf dem Gerät erfassten lokalen Gerätestatusmeldung, wie bisher).

Die Auswerteschaltung ist eine elektronische Komponente. Sie kann in Hardware und/oder Software ausgebildet sein. Die Auswerteschaltung kann z.B. als ein elektronischer Schaltkreis mit digitalen und /oder analogen Schaltungskomponenten ausgebildet sein, der eine Entscheidungslogik umfassen kann. Die Entscheidungslogik dient dazu, zu entscheiden, welche Priorität einer jeweiligen lokalen Gerätestatusmeldung in Bezug auf die anderen Gerätestatusmeldungen im gleichen Zeitraum zugeordnet werden soll bzw. welche Gerätestatusmeldung jeweils eine höhere und welche eine untergeordnete Priorität erhalten soll. Die Auswerteschaltung kann zur Auswertung auf ein Regelwerk zugreifen, das insbesondere in Form von Regeln in einer Datenbank oder in einem Speicher hinterlegt sein kann und ein Protokoll bzw. eine Policy für die Priorisierung von einer Menge von lokalen Gerätestatusmeldungen definiert. Die Auswerteschaltung kann automatisch aktiviert werden, wenn sich eine Zustandsänderung am Verbund der medizintechnischen Geräte, also an mindestens einem der Geräte, ergibt oder auch nach anderen vorkonfigurierbaren Ereignissen oder vorkonfigurierbaren Zeitintervallen.

Die Auswerteschaltung ist vorzugsweise einem bestimmten, medizintechnischen Gerät zugeordnet, das als Administratorgerät bzw. als Steueragent fungiert. Die Auswerteschaltung kann zentral in dem System oder lokal auf allen Geräten bereitgestellt werden und kann in Datenaustausch mit einem zentralen Server stehen, der Daten von allen oder ausgewählten medizintechnischen Geräten (z.B. von allen Dialysegeräten einer Dialysestation) sammelt und auswertet (z.B. mittels einer statistischen Auswertung). Es ist auch möglich, eine Auswerteschaltung für mehrere, medizintechnische Geräte auszubilden, die die Meldungen der Geräte dann je nach Geräteidentität auflösen kann (beispielsweise über einen entsprechenden Identifikationshinweis). Die Auswerteschaltung dient zur zentralen Auswertung aller lokalen Gerätestatusmeldungen in einem übereinstimmenden Zeitraum und zur Berechnung von priorisierten Warnmeldungen.

Der Steueragent ist eine elektronische Komponente und kann auf einem Computer oder auf einem medizintechnischen Gerät implementiert sein oder diesem zugeordnet sein. Der Steueragent fungiert in einer ersten Ausführungsform der Erfindung als zentrales Steuergerät für alle medizintechnischen Geräte des Verbundes, um für die Gesamtheit der Verbundgeräte auf konzertierte Weise eine priorisierte Liste von Warnmeldungen auf Basis der erfassten lokalen Gerätestatusmeldungen zu berechnen. Gemäß einer zweiten Ausführungsform der Erfindung fungiert der Steueragent dezentral und ist auf jedem der medizintechnischen Geräte installiert. In dieser zweiten Ausführungsform der Erfindung kann die Steuerung vollständig dezentral ausgeführt werden. Dies hat den technischen Vorteil, dass der Einsatz von zusätzlicher Hardware (Server, Mastergerät) nicht notwendig ist. So kann der Steueragent auf allen oder ausgewählten medizintechnischen Geräten implementiert werden. Alle (beteiligten) medizintechnischen Geräte senden und empfangen dann ihre jeweils aktuell anstehenden Status/Betriebszustände, und jedes Gerät (Maschine) kann aufgrund einer lokal hinterlegten Liste und einem Regelwerk entscheiden, ob der lokale (eigene) gemeldete Status der dringendste ist oder dessen Priorität in Bezug zu allen anderen (fremden) gemeldeten Stati zu berechnen. Es kann dann entsprechend das Versenden einer priorisierten Warnmeldung veranlassen.

Im Folgenden wird die Erfindung für ein Dialysegerät als Beispiel eines medizintechnischen Gerätes beschrieben, z.B. eines Hämodialysegerätes oder eines Peritonealdialysegerätes. Für den Fachmann liegt es jedoch auf der Hand, dass die Erfindung ebenso auf andere medizintechnische, computergesteuerte Geräte oder (Fluidmanagement-) Maschinen oder Blutbehandlungsgeräte angewendet oder übertragen werden kann, die Gerätemeldungen oder Gerätefehlermeldungen über eine Benutzerschnittstelle ausgeben.

In einer bevorzugten Weiterbildung der Erfindung wird die lokale Signalvorrichtung des jeweiligen medizintechnischen Gerätes mittels der berechneten, priorisierten Warnmeldungen modifiziert gesteuert. Der Begriff "modifiziert" bezieht sich auf zwei Aspekte: Zum einen wird die lokale Signalvorrichtung - anders als bisher - nicht mehr nur auf Basis von lokal erfassten Signalen des jeweiligen medizintechnischen Gerätes gesteuert, sondern auf Basis von Steuersignalen, die zentral oder lokal von dem Steueragenten berechnet und an die lokale Signalvorrichtung gesendet werden. Zum anderen erfolgt die Steuerung dynamisch in Abhängigkeit vom Zustand des Geräteverbundes mit allen medizintechnischen Geräten.

Gemäß einem weiteren Aspekt wird die Aufgabe der Erfindung gelöst durch einen Steueragenten zur konzertierten Berechnung von priorisierten Warnmeldungen für einen Verbund von medizintechnischen Geräten mit den Merkmalen des Patentanspruchs 1.

In einer bevorzugten Ausführungsform der Erfindung ist der Steueragent auf einem medizintechnischen Gerät und/oder auf einem zentralen Server implementiert. Dies hat den technischen Vorteil, dass keine zusätzliche Hardware bereitgestellt werden muss. Es ist auch möglich, ein dediziertes medizintechnisches Gerät mit der Funktion des Steueragenten zu belegen.

In einer weiteren, bevorzugten Ausführungsform der Erfindung ist die Eingangsschnittstelle zum Erfassen von allen lokalen Gerätestatusmeldungen von den medizintechnischen Geräten des Verbundes in einem konfigurierbaren Zeitintervall bestimmt.

Gemäß einem weiteren Aspekt wird die Aufgabe der Erfindung gelöst durch ein Verfahren zum koordinierten Steuern eines Verbundes von medizintechnischen Geräten in Hinblick auf die Ausgabe von Meldungen mit den Verfahrensschritten gemäß Patentanspruch 7.

In einer bevorzugten Ausführungsform der Erfindung ist es konfigurierbar, an welche Geräte des Verbundes die konzertiert berechnete, priorisierte Warnmeldung gesendet werden soll. So kann es beispielsweise eingestellt werden, dass die priorisierte Warnmeldung nur an diejenigen Geräte des Verbundes gesendet wird, von denen jeweils eine lokale Gerätestatusmeldung erfasst worden ist. Dies hat den Vorteil, dass fehlerfrei betriebene Geräte nicht mit unnötigen Meldungen belastet werden. Es kann aber auch wünschenswert sein, dass auf allen Geräten des Verbundes eine Anzeige erfolgt. Damit hat der Anwender mit einem Blick auf nur ein Gerät automatisch alle anderen Geräte auch im Blick (bzw. wird über deren Status informiert). Ebenso ist es möglich, auf den Geräten, auch priorisierte Warnmeldungen von anderen "Fremdgeräten" auszugeben. Insbesondere können die priorisierten Warnmeldungen von anderen Fremdgeräten ausgeben werden, die höher priorisiert sind, damit der Anwender sofort Handlungsbedarf auf einem Fremdgerät erkennen kann.

In einer weiteren bevorzugten Ausführungsform wird der jeweiligen (jeweils berechneten) priorisierten Warnmeldung ein Ausgabemodus zugeordnet. Der Ausgabemodus kann insbesondere ein optisches, akustisches Warnsignal und/oder ein Warnsignal in einem anderen Format umfassen, wobei die priorisierte Warnmeldung auf der lokalen Signalvorrichtung entsprechend dem jeweiligen Ausgabemodus ausgegeben wird. Der Ausgabemodus kann z.B. bei einem optischen Format auf einem Monitor des medizintechnischen Gerätes, insbesondere auf einem Touchscreen-Monitor, ausgegeben werden. Für die Zuordnung kann auf eine in einem Speicher hinterlegte Policy (Protokoll, optional mit Zugriff auf ein Regelwerk) zugegriffen werden, das z.B. vorsehen kann, dass eine priorisierte Warnmeldung mit höchster Prioritätsstufe immer mit einem roten Blinksignal und zusätzlich mit einer akustischen Ausgabe als Ausgabemodus ausgegeben werden muss, während ein niederpriorisiertes Warnsignal lediglich in optischer Form ausgegeben wird. Vorteilhafterweise kann die Policy für die Zuordnung des Ausgabemodus zu der Prioritätsstufe der Warnsignalmeldung auch während des Betriebs des Systems verändert werden. Die Änderung kann vorteilhafterweise zudem einheitlich für alle Geräte ausgeführt werden.

In einer anderen bevorzugten Ausführungsform der Erfindung kann die ursprünglich erfasste, lokale Gerätestatusmeldung zusätzlich zu der priorisierten Warnmeldung auf dem Gerät angezeigt werden. Damit ist es möglich, lokal (am Gerät) zu signalisieren, dass die lokale Gerätestatusmeldung aufgrund der Zustandsdaten der anderen Geräte des Verbundes verändert (herauf- oder herabgestuft) werden musste.

In einer weiteren, vorteilhaften Ausführungsform der Erfindung kann ausgewählten lokalen Gerätestatusmeldungen ein Sperrvermerk zugeordnet werden, der kennzeichnet, dass die jeweilige Gerätestatusmeldung bei der Berechnung der priorisierten Warnmeldung nicht heruntergestuft werden darf. Damit kann die Sicherheit für wichtige Meldungen erhöht werden, indem sichergestellt wird, dass deren Priorität nicht verringert wird.

In einer anderen, vorteilhaften Ausführungsform der Erfindung wird das Berechnen der priorisierten Warnmeldungen durch eine korrelative Verhandlung der Dringlichkeit aller erfassten lokalen Gerätestatusmeldungen auf Basis eines vorkonfigurierbaren Regelwerks ausgeführt. Das Regelwerk kann auf dem Steueragenten oder an einem anderen zentralen, über einen Netzwerkzugriff zugänglichen Speicherort oder auch lokal gespeichert sein. Vorteilhafterweise kann das Regelwerk auch während des Betriebs geändert und an die jeweiligen medizinischen Erfordernisse (z.B. Behandlung von Notfallpatienten) angepasst werden.

Gemäß einer weiteren, vorteilhaften Ausführungsform der Erfindung wird das modifizierte, koordinierte Steuern des Geräteverbundes nicht auf Basis der lokalen Gerätestatusmeldung, sondern auf Basis der in Relation zu allen Gerätestatusmeldungen priorisierten Warnmeldung ausgeführt.

Gemäß einer weiteren, vorteilhaften Ausführungsform der Erfindung ist das modifizierte, koordinierte Steuern des medizintechnischen Gerätes aktivierbar und de-aktivierbar. Alternativ oder kumulativ kann das modifizierte, koordinierte Steuern automatisch nach Eintreten eines vordefinierbaren Ereignisses ausgelöst werden. In vorkonfigurierbaren medizinischen Sonderfällen (z.B. bei der Behandlung von Notfallpatienten) kann auf die Ausgabe der lokalen Gerätestatusmeldung umgeschaltet werden - also auf das bisherige unkoordinierte Verfahren, ohne dass eine Priorisierung seitens des Steueragenten ausgeführt wird. Dies kann insbesondere dann hilfreich sein, wenn die Netzwerkverbindung unterbrochen oder fehlerbehaftet ist oder nicht die notwendige Übertragungsbandbreite zur Verfügung stellen kann. Dazu ist es in einer bevorzugten Ausführungsform der Erfindung vorgesehen, dass die Netzwerkverbindung zwischen den medizintechnischen Geräten und/oder dem Steueragenten automatisch überprüft wird und dass bei Erfassen eines Fehlers oder bei nicht ausreichender Verbindungsqualität ein Fehlersignal erzeugt wird. In Antwort auf das Fehlersignal wird dann der Betrieb von der koordinierten Steuerung wieder auf die herkömmliche, lokale Steuerung umgeschaltet. Dies kann vorzugsweise automatisch erfolgen. In diesem Fall ist das vordefinierbare Ereignis also eine fehlerhafte oder nicht ausreichende Netzwerkverbindung (oder Schnittstelle) und basiert auf einem automatischen Überprüfen der Netzwerkverbindung oder der jeweiligen Schnittstellen.

Gemäß einer weiteren, vorteilhaften Ausführungsform der Erfindung werden alle berechneten priorisierten Warnmeldungen für jedes medizintechnische Gerät aufgeschlüsselt gespeichert und/oder auf einer Ausgabeeinheit ausgegeben. Dies erhöht vorteilhafterweise die Transparenz und der Anwender erhält lokal an "seinem" Gerät Information über den aktuellen Zustand der anderen Geräte des Verbundes.

Gemäß einer vorteilhaften Weiterbildung der Erfindung umfasst das Verfahren folgende Verfahrensschritte:
- Erfassen einer Anwenderidentität, die jeweils spezifisch für einen Anwender des jeweiligen medizintechnischen Gerätes ist und/oder
- Erfassen eines Aufenthaltsortes des Anwenders des jeweiligen medizintechnischen Gerätes,
- wobei für das Berechnen der priorisierten Warnmeldung für das jeweilige medizintechnische Gerät die erfasste Anwenderidentität und/oder der erfasste Aufenthaltsort verrechnet werden.

Gemäß einer vorteilhaften Weiterbildung der Erfindung umfasst das Verfahren folgenden Verfahrensschritt, falls für mehrere medizintechnische Geräte hochpriorisierte Warnmeldungen berechnet worden sind:
- Erzeugen eines Anforderungsbefehls und Versenden desselben an vorkonfigurierte Instanzen über vorkonfigurierte Datenübertragungskanäle.

Der Anforderungsbefehl kann eine Nachricht umfassen, die signalisiert, dass zusätzliches Personal erforderlich ist und von anderen Stationen angefordert werden muss. In einer Weiterbildung kann der Anforderungsbefehl automatisch an weitere Instanzen zur Bereitstellung des Personals weitergeleitet werden.

Falls für mehrere medizintechnische Geräte hochpriorisierte Warnmeldungen berechnet worden sind, kann auf eine Kollisionsprüfungseinheit zugegriffen werden. Die Kollisionsprüfungseinheit ist dazu bestimmt, Prioritätskonflikte aufzulösen. Dazu können gemäß dem hinterlegten Regelwerk weitere Parameter abgefragt werden (z.B. Dauer und/oder Art der erfassten lokalen Statusmeldung), um eine Abstufung der Priorität zu erreichen.

Eine weitere Aufgabenlösung besteht in einem Computerprogrammprodukt, das in einen Speicher eines Computers oder eines elektronischen oder medizintechnischen Gerätes geladen oder ladbar ist mit einem Computerprogramm zur Durchführung des oben näher beschriebenen Verfahrens, wenn das Computerprogramm auf dem Computer oder dem elektronischen oder medizintechnischen Gerät ausgeführt wird.

Eine weitere Aufgabenlösung sieht ein Computerprogramm vor zur Durchführung aller Verfahrensschritte des oben näher beschriebenen Verfahrens, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

- Fig. 1: zeigt in einer schematischen Darstellung einen Verbund von Dialysegeräten im Datenaustauch mit einem Steueragenten gemäß einer vorteilhaften Ausführungsform der Erfindung.
- Fig. 2: zeigt weitere Bauteile der lokalen Signalvorrichtung und des Steueragenten und repräsentiert den Datenaustauch zwischen dem Dialysegerät und dem Steueragenten.
- Fig. 3: zeigt auf schematische Weise den Datenaustausch zwischen Dialysegerät und Steueragent gemäß einer ersten Ausführungsform der Erfindung und
- Fig. 4: zeigt den Datenaustauch gemäß einer zweiten Ausführungsform der Erfindung.
- Fig. 5: ist ein Ablaufdiagramm eines Verfahrens gemäß einer bevorzugten Ausführungsform der Erfindung.

### Detaillierte Beschreibung der Figuren

Im Folgenden wird die Erfindung anhand von Ausführungsbeispielen unter Bezugnahme auf die Figuren näher beschrieben.

Die Erfindung betrifft die koordinierte Steuerung eines Verbundes V von medizintechnischen Geräten, insbesondere Dialysegeräten D, in Hinblick auf die Ausgabe von Meldungen. Dazu umfasst jedes Dialysegerät D eine lokale Signalvorrichtung 10, die dazu bestimmt ist, Meldungen zum Gerätezustand auszugeben. Da mehrere Dialysegeräte D in einer Dialysestation betrieben werden, sind die Geräte D zu einem Verbund V zusammengefasst.

In der Praxis kommt es häufig vor, dass in einer Zeitphase mehrere Dialysegeräte D Meldungen ausgeben, weil sie sich in einem meldebedürftigen Zustand (z.B. Fehlerzustand) befinden. In diesem Fall kann mit den Vorkehrungen der Erfindung sichergestellt werden, dass die parallel ausgegebenen Meldungen priorisiert bzw. nach Dringlichkeit gewichtet werden, um dem Anwender eine effizientere Handhabung zu ermöglichen. Anhand der Priorisierung wird ihm ein Handlungs- bzw. Abarbeitungsschema vorgegeben und er weiß in welcher Reihenfolge er die Dialysegeräte zu bedienen hat. So kann er insgesamt für den Geräteverbund V schneller einen fehlerfreien Gesamtzustand herstellen.

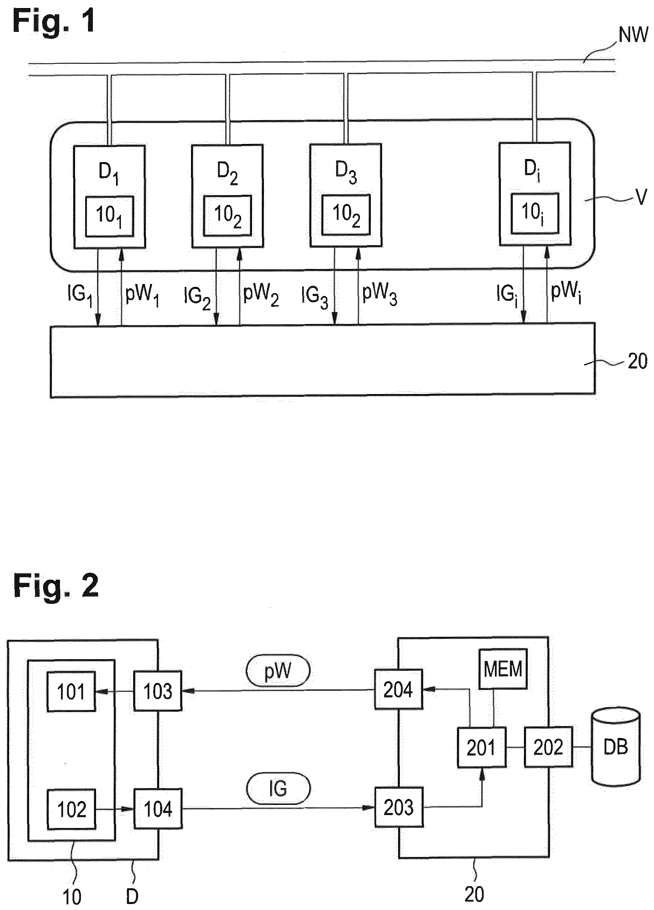

Wie in **Fig. 1** gezeigt, umfasst der Verbund V in diesem Beispiel die Dialysegeräte D₁, D₂, D₃ und Dᵢ mit deren lokalen Signalvorrichtungen 10₁, 10₂, 10₃, 10ᵢ. Die Dialysegeräte D stehen in Datenaustausch mit einem Steueragenten 20, der zur koordinierten Verarbeitung der lokalen Gerätestatusmeldungen lG bestimmt ist. Dazu werden die lokal erfassten lokalen Gerätestatusmeldungen lG gesammelt und ausgewertet und nach einem vorkonfigurierbaren Schema (Policy) priorisiert. Insbesondere wird die Dringlichkeit der jeweiligen parallelen Meldungen in Bezug zueinander ausgewertet. Dazu kann der Steueragent auf die in einer Datenbank DB abgelegten Policy zugreifen. Die berechneten priorisierten Warnmeldungen pW werden dann als Nachricht oder Nachrichtenkonvolut an das jeweilige Dialysegeräte D zur dortigen lokalen Ausgabe gesendet. Die berechneten priorisierten Warnmeldungen pW sind vorzugsweise als elektronischer Datensatz ausgebildet.

Die Dialysegeräte D können untereinander über ein Netzwerk NW in Datenaustausch stehen. Dies ist jedoch nicht zwingend erforderlich für die Umsetzung der Erfindung und von daher nur fakultativ.

**Fig. 2** zeigt eines der Dialysegeräte D des Verbundes V und den Steueragenten 20 mit weiteren Bauteilen und den Datenaustausch zwischen den Geräten. Die lokale Signalvorrichtung 10 umfasst üblicherweise eine Ausgabeeinheit 101, die z.B. als optische und/oder akustische Ausgabeeinheit zur Ausgabe von Meldungen und insbesondere von priorisierten Warnmeldungen pW ausgebildet sein kann, und eine Erfassungseinheit 102, die zur Erfassung der lokalen Gerätestatusmeldung lG bestimmt ist. Die lokale Signalvorrichtung 10 kann eine Eingangsschnittstelle 103 und eine Ausgangsschnittstelle 104 aufweisen. Über die Ausgangsschnittstelle 104 wird die lokal erfasste lokale Gerätestatusmeldung lG an den Steueragenten 20 gesendet. Über die Eingangsschnittstelle 103 empfängt die lokale Signalvorrichtung 10 die berechnete priorisierte Warnmeldung pW von dem Steueragenten 20, um diese auf der Ausgabeeinheit 101 auszugeben.

Der Steueragent 20 umfasst eine Erfassungsschnittstelle 203, über die er die lokalen Gerätestatusmeldungen lG der Dialysegeräte D des Verbundes V empfängt und die mit einem Prozessor 201 mit einer Auswerteschaltung in Verbindung steht. Der Prozessor 201 berechnet aus allen konsolidierten lokalen Gerätestatusmeldungen lG der Dialysegeräte D des Verbundes V eine Menge von priorisierten Warnmeldungen und insbesondere ein priorisierte Warnmeldung pW für jedes Dialysegerät D, die er über die Signalausgangsschnittstelle 204 an die lokale Signalvorrichtung 10 sendet. Der Prozessor 201 kann zur konzertierten Berechnung der priorisierten Warnmeldungen pW für den Geräteverbund V über eine Datenbank-Schnittstelle 202 auf eine Datenbank DB zugreifen, in welcher Regeln als Policy zur Auswertung der lokalen Gerätestatusmeldungen lG hinterlegt sind.

Der Steueragent 20 kann einen Speicher MEM umfassen, in welchem Zuordnungen hinterlegt sein können. So kann für jedes Dialysegerät D, die für ihn spezifisch berechnete priorisierte Warnmeldung pW mit einer spezifischen Priorität abgelegt werden. Die Priorität repräsentiert dabei die Dringlichkeit der jeweiligen lokalen Gerätestatusmeldung lG im Kontext aller anderen lokalen Gerätestatusmeldungen (der anderen Dialysegeräte D). In diesem Fall kann folgende Zuordnung hinterlegt sein:

| | | |
|---|---|---|
| D₁ | pW₁ | Prio 1 |
| D₂ | pW₂ | Prio 3 |
| D₃ | pW₃ | Prio 1 |
| D₄ | pW₄ | Prio 2 |
| D₅ | pW₅ | Prio 3 |

Listet man die Geräte D nach der Dringlichkeit ihrer Meldungen so ergibt sich in obigem Beispiel folgende Reihenfolge: D₁/D₃ - D₄ - D₂/D₅. Die Geräte 1 und 3 sowie 2 und 5 haben jeweils dieselbe Priorität. In Fall einer Prioritätsübereinstimmung können weitere Parameter abgefragt werden, um den jeweiligen Gerätezustand noch detaillierter bestimmen zu können und eine weitere Differenzierung bereitstellen zu können.

Zusätzlich kann in dem Speicher MEM zu den in obiger Tabelle aufgelisteten Parametern ein Ausgabemodus zugeordnet werden. Der Ausgabemodus kennzeichnet, in welcher Form die priorisierte Warnmeldung pW auf der lokalen Signalvorrichtung 10 ausgegeben werden soll. Hier kann z.B. die Dauer der Meldung, das Format (optisch und/oder akustisch), mit oder ohne Blinkmodus, die Lautstärke bzw. Helligkeit etc. eingestellt werden. Der Ausgabemodus kann von Gerät zu Gerät unterschiedlich eingestellt werden. Er kann auch für jede Priorität unterschiedlich vorkonfiguriert sein.

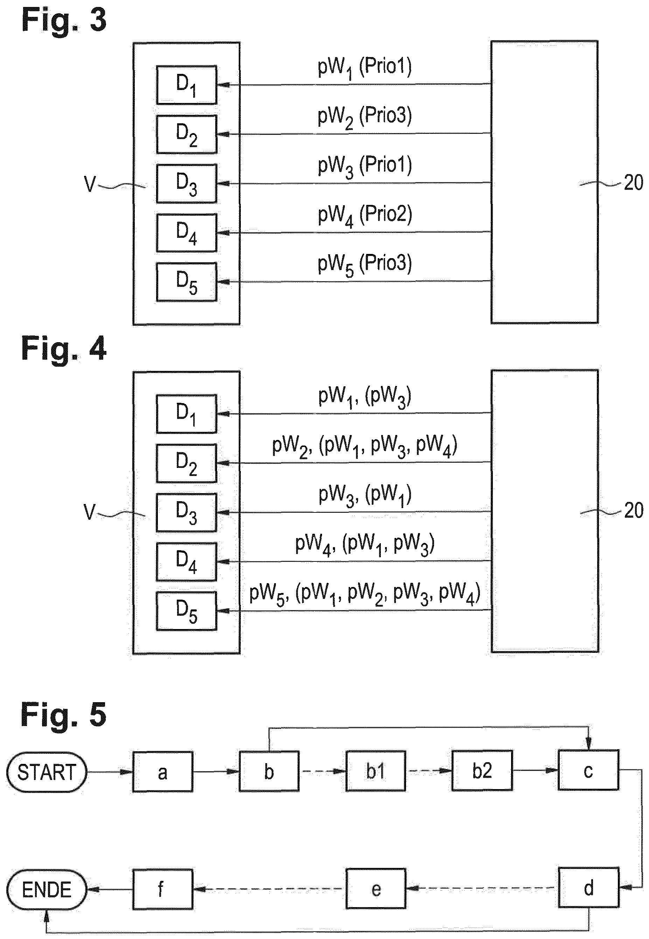

In einer ersten Ausführungsform der Erfindung kann das System in einem Singularmodus betrieben werden, der in **Fig. 3** beispielhaft dargestellt ist. In dem Singularmodus wird nur die eine für das jeweilige Dialysegerät berechnete, priorisierte Warnmeldung pW an das Dialysegerät D gesendet. Die berechneten priorisierten Warnmeldungen pW entsprechen denjenigen von dem in der obigen Tabelle dargestellten Beispiel. An das erste Gerät D₁ wird somit nur die das erste Gerät betreffende priorisierte Warnmeldung pW₁ (mit Priorität 1) gesendet, an das zweite Gerät D₂ wird somit nur die das zweite Gerät betreffende priorisierte Warnmeldung pW₂ (mit Priorität 3) gesendet... an das fünfte Gerät D₅ wird somit nur die das fünfte Gerät betreffende priorisierte Warnmeldung pW₅ (mit Priorität 3) gesendet. Wird das System in diesem Singularmodus betrieben, kann das zwischen den Geräten D und dem Steueragenten 20 übertragende Datenvolumen verringert werden.

**Fig. 4** zeigt eine zweite Ausführungsform der Erfindung, bei der das System in einem Verbundmodus betrieben wird. In dem Verbundmodus wird von dem Steueragenten 20 nicht nur die priorisierte Warnmeldung pW an das Dialysegerät D übertragen, die das jeweilige Dialysegerät D betrifft, sondern es werden auch alle oder ausgewählte fremde, priorisierte Warnmeldungen pW für Fremdgeräten (also fremde, andere Dialysegeräte D des Verbundes V) an das jeweilige Dialysegerät D übertragen. In einer bevorzugten Ausführungsform der Erfindung werden nur die priorisierten Warnmeldungen mit gleicher oder höherer Priorität übertragen. Dies erfordert zwar ein höheres Datenvolumen, das vom Steueragenten 20 an die lokale Signalvorrichtung übertragen werden muss, ermöglicht jedoch lokal an dem jeweiligen Dialysegerät D mehr Aufschluss über die Gesamtsituation des Verbundes V. So kann ein Anwender, der an einem zweiten Dialysegerät D₂ arbeitet, auf dem die priorisierte Warnmeldung pW₂ mit Priorität 3 (relativ geringe Dringlichkeit) ausgegeben wird, darüber informiert werden, dass auf dem ersten und dritten Dialysegerät D₁, D₃ ein höchstpriorisierte Warnmeldung mit Priorität 1 ausgegeben wird. Dies ermöglicht ihm, zu überprüfen, ob er kurzfristig seinen Arbeitsplatz wechseln kann, um die Meldung auf dem ersten oder dritten Gerät zu bearbeiten. Entsprechend wird der Anwender an dem fünften Dialysegerät D₅ informiert, dass auf dem fremden, vierten Dialysegerät D₄, dem die vierte priorisierte Warnmeldung pW₄ zugeordnet ist, deren Priorität 2 beträgt. Für den Fachmann liegt es auf der Hand, dass es möglich ist, nicht die fremden priorisierten Warnmeldungen pW (die für andere Geräte bestimmt sind und eine gleiche oder höhere Priorität haben) zu übertragen, sondern nur einen Hinweis auf das jeweilige Dialysegerät D, dem die Meldung pW zugeordnet ist.

In einer vorteilhaften Weiterbildung der Erfindung kann zusätzlich zu den Prioritätssignalen von fremden Geräten D des Verbundes V auch deren Position auf einer grafischen Karte angezeigt werden, so dass der Anwender unmittelbar darüber informiert wird, wo sich die Geräte D mit der jeweiligen hoch priorisierten Warnmeldung befinden. Diese Weiterbildung kann vorteilhafterweise in dem Verbundmodus angewendet werden.

**Fig. 5** zeigt ein Ablaufdiagramm für das Verfahren gemäß einem Ausführungsbeispiel der Erfindung. Nach dem Start des Verfahrens erfolgt in Schritt a das Erfassen der lokalen Gerätestatusmeldungen lG auf allen oder ausgewählten Geräten des Verbundes V. In Schritt b werden die erfassten lokalen Gerätestatusmeldungen lG gesammelt und auf koordinierte Weise konsolidiert, vorzugsweise auf dem Steueragenten 20. Daraufhin werden die konsolidierten lokalen Gerätestatusmeldungen lG in Schritt c - vorzugsweis aber nicht notwendegerweise zentral - ausgewertet, um eine Menge von priorisierten Warnmeldungen pW zu generieren, bei denen der Gesamtzustand aller Geräte des Verbundes verrechnet und berücksichtigt wird. In Schritt d können die berechneten priorisierten Warnmeldungen pW an die Dialysegeräte D zur Ausgabe gesendet werden.

Wie in Fig. 5 mit den gepunkteten Pfeilen dargestellt kann in einer möglichen Weiterbildung der Erfindung nach dem Schritt b der Schritt b1 ausgeführt werden, bei dem eine Anwenderidentität des Anwenders des Dialysegerätes D erfasst wird. Es ist auch möglich, in Schritt b2 einen Aufenthaltsort des Anwenders zu erfassen. Je nachdem welche Parameter erfasst worden sind, werden diese bei der Berechnung der priorisierten Warnmeldung pW berücksichtigt.

In einer vorteilhaften Ausführungsform der Erfindung kann in Schritt e jeder priorisierten Warnmeldung pW ein Ausgabemodus zugeordnet werden. Der Ausgabemodus kennzeichnet die Art der Ausgabe auf der lokalen Signalvorrichtung 10. Der Ausgabemodus kann eine optische, akustische und/oder eine Ausgabe eines Warnsignals in einem anderen Format kennzeichnen, wobei die priorisierte Warnmeldung pW auf der lokalen Signalvorrichtung 10 oder auf einer Ausgabeeinheit des Dialysegerätes (z.B. einem Monitor) ausgegeben wird.

In einer weiteren Ausführungsform der Erfindung kann in Schritt f ein Anforderungsbefehl erzeugt werden. Der Anforderungsbefehl kann z.B. signalisieren, dass weiteres Fachpersonal zur Bearbeitung der parallel ausgegebenen priorisierten Warnmeldungen pW erforderlich ist. Der Anforderungsbefehle kann das Fachpersonal beispielsweise gleich von dem erfassen Aufenthaltsort angefordert werden. Dabei ist es möglich, die Qualifikation des Fachpersonals zu berücksichtigen, um z.B. höher qualifiziertes Personal für hoch priorisierte Warnmeldungen zu buchen. Der Aufenthaltsort kann z.B. über Arbeitspläne und/oder Zeiterfassungssysteme ermittelt werden. Alternativ können Pager zur Lokalisierung verwendet werden, die die Person mit sich trägt und deren Signale erfasst werden. Der Anforderungsbefehl kann an weitere computer-basierte Instanzen (z.B. fremde Dialysegeräte D oder an Pager, die das Fachpersonal mit sich trägt) oder mittels anderer Nachrichtenformate, wie Email oder SMS) weitergeleitet werden, um an einem entfernten Ort zu signalisieren, dass Handlungsbedarf besteht.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung alternativ oder kumulativ zur Ausgabeeinheit der lokalen Signalvorrichtung andere Ausgabeeinheiten, z.B. die grafische Benutzeroberfläche des medizinischen Gerätes bereitzustellen, auf denen die priorisierte Warnmeldung pW ausgegeben wird. Für einen Fachmann ist es insbesondere offensichtlich, dass die Erfindung nicht nur für Dialysegeräte angewendet werden kann, sondern auch für andere medizintechnische Geräte D, bei denen ein Gerätestatus überwacht werden soll und die in einem Verbund V von Geräten betrieben werden. Bei den Meldungen kann es sich um Fehlermeldungen, aber auch um andere Meldungen in Bezug auf einen Gerätezustand handeln.

Des Weiteren können die Bauteile des medizinischen Systems zur koordinierten Meldungsbehandlung bzw. zur Fehlerbehandlung auf mehrere physikalische Produkte verteilt realisiert werden.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHEN

- D: Medizintechnisches Gerät, insbesondere Dialysegerät
- V: Verbund von medizintechnischen Geräten

- 10: Lokale Signalvorrichtung
- 101: Ausgabeeinheit
- 102: Erfassungseinheit für lokale Gerätestatusmeldung
- 103: Eingangsschnittstelle
- 104: Ausgangsschnittstelle

- 20: Steueragent
- 201: Mikroprozessor mit Auswerteschaltung
- 202: Datenbankschnittstelle
- 203: Erfassungsschnittstelle
- 204: Signalausgangsschnittstelle

- DB: Datenbank
- NW: Netzwerk

- lG: Lokale Gerätestatusmeldung
- pW: Priorisierte Warnmeldung

- a: Erfassen der lokalen Gerätestatusmeldung
- b: Sammeln und Konsolidieren der erfassten lokalen Gerätestatusmeldungen
- b1: Erfassen einer Anwenderidentität
- b2: Erfassen eines Aufenthaltsortes
- c: Auswerten der gesammelten lokalen Gerätestatusmeldungen
- d: Überragen der priorisierten Warnmeldungen an die medizintechnischen Geräte
- e: Zuordnen eines Ausgabemodus
- f: Erzeugen eines Anforderungsbefehls

## Patentansprüche

1. Steueragent (20) zur Berechnung von priorisierten Warnmeldungen (pW) für einen Verbund (V) von medizintechnischen Geräten (D), insbesondere Dialysegeräten, mit:
- einer Eingangsschnittstelle (203), die zum Erfassen von lokalen Gerätestatusmeldungen (lG) von den medizintechnischen Geräten (D) des Verbundes (V) bestimmt ist, wobei eine erfasste lokale Gerätestatusmeldung (lG) einen Zustand des jeweiligen medizintechnischen Gerätes (D) oder der an das jeweilige medizintechnische Gerät (D) angeschlossenen technischen Vorrichtungen betrifft, und wobei die erfasste lokale Gerätestatusmeldung (lG) eine Fehlermeldung, eine Warnung, ein Notfallsignal und/oder eine Alarmmeldung umfasst;
- eine Auswerteschaltung, die zur Auswertung der erfassten lokalen Gerätestatusmeldungen (lG) und zur konzertierten Berechnung von priorisierten Warnmeldungen (pW) für den Verbund (V) von medizintechnischen Geräten (D) bestimmt ist und die weiterhin dazu ausgebildet ist, die berechneten, priorisierten Warnmeldungen (pW) über eine Ausgangsschnittstelle (204) an die medizintechnischen Geräte (D) des Verbundes (V) zur modifizierten Steuerung des jeweiligen medizintechnischen Gerätes (D) zu versenden, wobei das Berechnen der priorisierten Warnmeldungen (pW) durch eine korrelative Verhandlung der Dringlichkeit aller erfassten lokalen Gerätestatusmeldungen (lG) auf Basis eines vorkonfigurierbaren Regelwerks ausgeführt wird;
wobei die lokale Gerätestatusmeldung (lG) zusätzlich zu der priorisierten Warnmeldung (pW) ausgegeben wird, um eine Änderung der lokalen Gerätestatusmeldung (lG) zu repräsentieren, wobei die lokale Gerätestatusmeldung (lG) aufgrund von Zustandsdaten der anderen medizintechnischen Geräte (D) des Verbundes (V) verändert, insbesondere heraufgestuft oder herabgestuft, wurde.

2. Steueragent (20) nach dem unmittelbar vorangehenden Anspruch, **dadurch gekennzeichnet, dass** der Steueragent (20) auf einem medizintechnischen Gerät (D) oder auf einem zentralen Server implementiert ist.

3. Steueragent (20) nach einem der vorangehenden Ansprüche, wobei der Zustand des jeweiligen medizintechnischen Gerätes (D) mittelbar errechnet wird aus zumindest einem in der jeweiligen lokalen Gerätestatusmeldung (D) umfassten Sensorsignal und/oder anderen technischen Betriebsparameter des medizintechnischen Gerätes (D).

4. Steueragent (20) nach einem der vorangehenden Ansprüche, **dadurch gekennzeichnet, dass** die Eingangsschnittstelle (203) zum Erfassen von allen lokalen Gerätestatusmeldungen (lG) von den medizintechnischen Geräten (D) des Verbundes (V) in einem konfigurierbaren Zeitintervall oder nach Eintritt eines konfigurierbaren Ereignisses bestimmt ist.

5. Vernetztes Statusmeldungssystem für einen Verbund (V) von medizintechnischen Geräten (D), insbesondere Dialysegeräten, mit:
- einer Vielzahl von medizintechnischen Geräten (D), wobei jedes medizintechnische Gerät (D) eine lokale Signalvorrichtung (10) umfasst, die zum Erfassen einer lokalen Gerätestatusmeldung (lG) bestimmt ist und in Datenaustausch steht mit einem Steueragenten (20) gemäß einem der vorangehenden Steueragentenansprüche; und
- dem Steueragenten (20).

6. Vernetztes Statusmeldungssystem nach dem unmittelbar vorangehenden Statusmeldungssystemanspruch, bei dem die lokale Signalvorrichtung (10) des jeweiligen medizintechnischen Gerätes (D) mittels der berechneten, priorisierten Warnmeldungen (pW) modifiziert gesteuert wird.

7. Verfahren zum koordinierten Steuern eines Verbundes (V) von medizintechnischen Geräten (D), insbesondere Dialysegeräten, in Hinblick auf die Ausgabe von Meldungen, wobei das Verfahren folgende Verfahrensschritte umfasst:
- Erfassen (a) von lokalen Gerätestatusmeldungen (lG) von medizintechnischen Geräten (D) des Verbundes (V), wobei eine erfasste lokale Gerätestatusmeldung (lG) einen Zustand des jeweiligen medizintechnischen Gerätes (D) oder der an das jeweilige medizintechnische Gerät (D) angeschlossenen technischen Vorrichtungen betrifft, und wobei die erfasste lokale Gerätestatusmeldung (lG) eine Fehlermeldung, eine Warnung, ein Notfallsignal und/oder eine Alarmmeldung umfasst;
- Sammeln und Konsolidieren (b) von allen erfassten, lokalen Gerätestatusmeldungen (lG);
- Auswerten (c) aller gesammelten und konsolidierten erfassten, lokalen Gerätestatusmeldungen, um für alle medizintechnischen Geräte (D) des Verbundes priorisierte Warnmeldungen (pW) auf konzertierte Weise zu berechnen, wobei das Berechnen der priorisierten Warnmeldungen (pW) durch eine korrelative Verhandlung der Dringlichkeit aller erfassten lokalen Gerätestatusmeldungen (lG) auf Basis eines vorkonfigurierbaren Regelwerks ausgeführt wird;
- Übertragen (d) der für das jeweilige medizintechnische Gerät (D) berechneten priorisierten Warnmeldung (pW) an das jeweilige medizintechnische Gerät (D) zum modifizierten, koordinierten Steuern der medizintechnischen Geräte (D) des Verbundes (V) auf Basis der übertragenen priorisierten Warnmeldung (pW);
wobei die lokale Gerätestatusmeldung (lG) zusätzlich zu der priorisierten Warnmeldung (pW) ausgegeben wird, um eine Änderung der lokalen Gerätestatusmeldung (lG) zu repräsentieren, wobei die lokale Gerätestatusmeldung (lG) aufgrund von Zustandsdaten der anderen medizintechnischen Geräte (D) des Verbundes (V) verändert, insbesondere heraufgestuft oder herabgestuft, wurde.

8. Verfahren nach dem unmittelbar vorangehenden Verfahrensanspruch, **dadurch gekennzeichnet, dass** das Auswerten zentral oder lokal auf jedem der medizintechnischen Geräte (D) ausgeführt wird.

9. Verfahren nach einem der vorangehenden Verfahrensansprüche, wobei der Zustand des jeweiligen medizintechnischen Gerätes (D) mittelbar errechnet wird aus zumindest einem in der jeweiligen lokalen Gerätestatusmeldung (D) umfassten Sensorsignal und/oder anderen technischen Betriebsparameter des medizintechnischen Gerätes (D).

10. Verfahren nach einem der vorangehenden Verfahrensansprüche, **dadurch gekennzeichnet, dass** für jeweils jede der priorisierten Warnmeldungen (pW) ein Ausgabemodus berechnet und zugeordnet (e) wird, wobei der Ausgabemodus die Ausgabe eines optischen, akustischen Warnsignals und/oder eines Warnsignals in einem anderen Format umfasst, so dass die priorisierte Warnmeldung (pW) auf der lokalen Signalvorrichtung (10) oder auf einer Ausgabeeinheit in dem Ausgabemodus ausgegeben wird.

11. Verfahren nach einem der vorangehenden Verfahrensansprüche, **dadurch gekennzeichnet, dass** ausgewählten lokalen Gerätestatusmeldungen (lG) ein Sperrvermerk zugeordnet werden kann, der kennzeichnet, dass die jeweilige Gerätestatusmeldung nicht durch die berechnete priorisierte Warnmeldung (pW) abgestuft werden darf.

12. Verfahren nach einem der vorangehenden Verfahrensansprüche, **dadurch gekennzeichnet, dass** das modifizierte, koordinierte Steuern nicht auf Basis der lokalen Gerätestatusmeldung (lG), sondern auf Basis der in Relation zu allen Gerätestatusmeldungen priorisierten Warnmeldung (pW) ausgeführt wird.

13. Verfahren nach einem der vorangehenden Verfahrensansprüche, **dadurch gekennzeichnet, dass** das modifizierte, koordinierte Steuern des medizintechnischen Gerätes (D) aktivierbar und de-aktivierbar ist oder automatisch nach Eintreten eines vordefinierbaren Ereignisses ausgelöst wird.

14. Verfahren nach einem der vorangehenden Verfahrensansprüche, **dadurch gekennzeichnet, dass** alle berechneten priorisierten Warnmeldungen (pW) für jedes medizintechnische Gerät (D) aufgeschlüsselt gespeichert und/oder ausgegeben werden.

15. Verfahren nach einem der vorangehenden Verfahrensansprüche, **dadurch gekennzeichnet, dass** das Verfahren folgende Verfahrensschritte umfasst:
- Erfassen (b1) einer Anwenderidentität, die jeweils spezifisch für einen Anwender des jeweiligen medizintechnischen Gerätes (D) ist und/oder
- Erfassen (b2) eines Aufenthaltsortes des Anwenders des jeweiligen medizintechnischen Gerätes (D),
- wobei für das Berechnen der priorisierten Warnmeldung (pW) für das jeweilige medizintechnische Gerät (D) die erfasste Anwenderidentität und/oder der erfasste Aufenthaltsort verrechnet werden.

16. Verfahren nach dem unmittelbar vorangehenden Verfahrensanspruch, **dadurch gekennzeichnet, dass** das Verfahren folgenden Verfahrensschritt umfasst, falls für mehrere medizintechnische Geräte (D) hochpriorisierte Warnmeldungen berechnet worden sind:
- Erzeugen (f) eines Anforderungsbefehls und Versenden desselben an vorkonfigurierte Instanzen über vorkonfigurierte Datenübertragungskanäle.

## Claims

1. Control agent (20) for calculating prioritized warning messages (pW) for a network (V) of medical devices (D), in particular dialysis devices, comprising:
- an input interface (203) intended for detecting local device status messages (IG) from the medical devices (D) of the network (V), wherein a detected local device status message (IG) relates to a state of the respective medical device (D) or the technical devices connected to the respective medical device (D), and wherein the detected local device status message (IG) comprises an error message, a warning, an emergency signal, and/or an alarm message;
- an evaluation circuit which is intended for evaluating the detected local device status messages (IG) and for the concerted calculation of prioritized warning messages (pW) for the network (V) of medical devices (D) and which is further designed to send the calculated, prioritized warning messages (pW) via an output interface (204) to the medical devices (D) of the network (V) for modified control of the respective medical device (D), wherein the calculation of the prioritized warning messages (pW) is performed by a correlative negotiation of the urgency of all recorded local device status messages (IG) on the basis of a preconfigurable set of rules;
wherein the local device status message (IG) is output in addition to the prioritized warning message (pW) in order to represent a change in the local device status message (IG), wherein the local device status message (IG) is changed, in particular upgraded or downgraded.

2. Control agent (20) according to the immediately preceding claim, **characterized in that** the control agent (20) is implemented on a medical device (D) or on a central server.

3. Control agent (20) according to one of the preceding claims, wherein the status of the respective medical device (D) is indirectly calculated from at least a sensor signal and/or other technical operating parameters of the medical device (D) included in the respective local device status message (D).

4. Control agent (20) according to one of the preceding claims, **characterized in that** the input interface (203) is designed to detect all local device status messages (IG) from the medical devices (D) of the network (V) at a configurable time interval or after the occurrence of a configurable event.

5. Networked status message system for a network (V) of medical devices (D), in particular dialysis devices, comprising:
- a plurality of medical devices (D), each medical device (D) comprising a local signal device (10) that is designed to detect a local device status message (IG) and exchanges data with a control agent (20) according to one of the preceding control agent claims; and
- the control agent (20).

6. Networked status message system according to the immediately preceding status message system claim, in which the local signaling device (10) of the respective medical device (D) is controlled in a modified manner by means of the calculated, prioritized warning messages (pW).

7. Method for the coordinated control of a network (V) of medical devices (D), in particular dialysis devices, with regard to the output of messages, wherein the method comprises the following method steps:
- Detecting (a) local device status messages (IG) from medical devices (D) in the network (V), wherein a detected local device status message (IG) relates to a state of the respective medical device (D) or of the technical devices connected to the respective medical device (D), and wherein the detected local device status message (IG) comprises an error message, a warning, an emergency signal, and/or an alarm message;
- Collecting and consolidating (b) all detected local device status messages (IG);
- Evaluating (c) all collected and consolidated detected local device status messages in order to calculate prioritized warning messages (pW) for all medical devices (D) in the network in a concerted manner, wherein the calculation of the prioritized warning messages (pW) is performed by a correlative negotiation of the urgency of all detected local device status messages (IG), and wherein the calculation of the prioritized warning messages (pW) is performed by a correlative negotiation of the urgency of all detected local device status messages (IG), and wherein the calculation of the prioritized warning messages (pW) is performed by a correlative negotiation of the urgency of all detected local device status messages (IG), and wherein the (pW) for all medical devices (D) in the network in a concerted manner, wherein the calculation of the prioritized warning messages (pW) is performed by a correlative negotiation of the urgency of all recorded local device status messages (IG) based on a preconfigurable set of rules;
- Transmission (d) of the prioritized warning message (pW) calculated for the respective medical device (D) to the respective medical device (D) for modified, coordinated control of the medical devices (D) of the network (V) based on the transmitted prioritized warning message (pW);
wherein the local device status message (IG) is output in addition to the prioritized warning message (pW) in order to represent a change in the local device status message (IG), wherein the local device status message (IG) is changed, upgraded, or downgraded based on status data from the other medical devices (D) in the network (V). in particular upgraded or downgraded.

8. Method according to the immediately preceding method claim, **characterized in that** the evaluation is performed centrally or locally on each of the medical devices (D).

9. Method according to one of the preceding method claims, wherein the status of the respective medical device (D) is calculated indirectly from at least one sensor signal and/or other technical operating parameters of the medical device (D) included in the respective local device status message (D).

10. Method according to one of the preceding method claims, **characterized in that** an output mode is calculated and assigned (e) for each of the prioritized warning messages (pW), wherein the output mode comprises the output of an optical, acoustic warning signal and/or a warning signal in another format, so that the prioritized warning message (pW) is output on the local signal device (10) or on an output unit in the output mode.

11. Method according to one of the preceding method claims, **characterized in that** selected local device status messages (IG) can be assigned a blocking note which indicates that the respective device status message must not be downgraded by the calculated prioritized warning message (pW).

12. Method according to one of the preceding method claims, **characterized in that** the modified, coordinated control is not performed on the basis of the local device status message (IG), but on the basis of the warning message (pW) prioritized in relation to all device status messages.

13. Method according to one of the preceding method claims, **characterized in that** the modified, coordinated control of the medical device (D) can be activated and deactivated or is triggered automatically after a predefined event occurs.

14. Method according to one of the preceding method claims, **characterized in that** all calculated prioritized warning messages (pW) are stored and/or output in a broken-down form for each medical device (D).

15. Method according to one of the preceding method claims, **characterized in that** the method comprises the following method steps:
- Detecting (b1) a user identity that is specific to a user of the respective medical device (D) and/or
- recording (b2) a location of the user of the respective medical device (D),
- wherein the recorded user identity and/or the recorded location are taken into account when calculating the prioritized warning message (pW) for the respective medical device (D).

16. Method according to the immediately preceding method claim, **characterized in that** the method comprises the following method step if high-priority warning messages have been calculated for several medical devices (D):
- Generating (f) a request command and sending it to preconfigured instances via preconfigured data transmission channels.

## Revendications

1. Agent de commande (20) pour calculer des messages d'alerte prioritaires (pW) pour un réseau (V) d'appareils médicaux (D), en particulier d'appareils de dialyse, comprenant :
- une interface d'entrée (203) destinée à détecter des messages d'état d'appareil locaux (IG) provenant des appareils médicaux (D) du réseau (V), un message d'état d'appareil local détecté (IG) concernant un état de l'appareil médical respectif (D) ou des dispositifs techniques raccordés à l'appareil médical (D) respectif, et le message d'état d'appareil local (IG) détecté comprenant un message d'erreur, un avertissement, un signal d'urgence et/ou un message d'alarme ;
- un circuit d'évaluation qui est destiné à l'évaluation des messages d'état d'appareil locaux détectés (IG) et au calcul concerté de messages d'avertissement prioritaires (pW) pour le réseau (V) d'appareils médicaux (D) et qui est en outre conçu pour envoyer les messages d'avertissement prioritaires calculés (pW) via une interface de sortie (204) aux appareils médicaux (D) du réseau (V) pour la commande modifiée de l'appareil médical (D) concerné, le calcul des messages d'alerte prioritaires (pW) est effectué par une négociation corrélative de l'urgence de tous les messages d'état d'appareil local (IG) enregistrés sur la base d'un ensemble de règles préconfigurables ;
le message d'état d'appareil local (IG) étant émis en plus du message d'alerte priorisé (pW) afin de représenter une modification du message d'état d'appareil local (IG), le message d'état d'appareil local (IG) étant modifié, en particulier reclassé à la hausse ou à la baisse.

2. Agent de commande (20) selon la revendication précédente, **caractérisé en ce que** l'agent de commande (20) est implémenté sur un appareil médical (D) ou sur un serveur central.

3. Agent de commande (20) selon l'une des revendications précédentes, l'état de l'appareil médical (D) concerné étant calculé indirectement à partir d'au moins un signal de capteur et/ou d'autres paramètres techniques de fonctionnement de l'appareil médical (D) compris dans le message d'état local de l'appareil (D) concerné.

4. Agent de commande (20) selon l'une des revendications précédentes, **caractérisé en ce que** l'interface d'entrée (203) est destinée à détecter tous les messages d'état locaux (IG) provenant des appareils médicaux (D) du réseau (V) à un intervalle de temps configurable ou après la survenue d'un événement configurable.

5. Système de message d'état en réseau pour un réseau (V) d'appareils médicaux (D), en particulier d'appareils de dialyse, comprenant :
- une pluralité d'appareils médicaux (D), chaque appareil médical (D) comprenant un dispositif de signalisation local (10) destiné à détecter un message d'état local (IG) et échangeant des données avec un agent de commande (20) selon l'une des revendications précédentes relatives à l'agent de commande ; et
- l'agent de commande (20).

6. Système de message d'état en réseau selon la revendication de système de message d'état immédiatement précédente, dans lequel le dispositif de signalisation local (10) de l'appareil médical (D) respectif est commandé de manière modifiée au moyen des messages d'avertissement calculés et priorisés (pW).

7. Procédé pour commander de manière coordonnée un réseau (V) d'appareils médicaux (D), en particulier d'appareils de dialyse, en vue de l'émission de messages, le procédé comprenant les étapes suivantes :
- la saisie (a) des messages d'état locaux (IG) des appareils médicaux (D) du réseau (V), un message d'état d'appareil local (IG) détecté concernant un état de l'appareil médical (D) respectif ou des dispositifs techniques raccordés à l'appareil médical (D) respectif, et le message d'état d'appareil local (IG) détecté comprenant un message d'erreur, un avertissement, un signal d'urgence et/ou un message d'alarme ;
- collecte et consolidation (b) de tous les messages d'état d'appareil local (IG) enregistrés ;
- Évaluation (c) de tous les messages d'état d'appareil locaux détectés, collectés et consolidés afin de calculer de manière concertée des messages d'avertissement prioritaires (pW) pour tous les appareils médicaux (D) du réseau, le calcul des messages d'avertissement prioritaires (pW) étant effectué par une négociation corrélative de l'urgence de tous les messages d'état d'appareil local (IG) enregistrés sur la base d'un ensemble de règles préconfigurables ;
- Transmission (d) du message d'alerte priorisé (pW) calculé pour l'appareil médical (D) concerné à l'appareil médical (D) concerné afin de commander de manière modifiée et coordonnée les appareils médicaux (D) du réseau (V) sur la base du message d'alerte priorisé (pW) transmis ;
le message d'état local de l'appareil (IG) étant émis en plus du message d'alerte prioritaire (pW) afin de représenter une modification du message d'état local de l'appareil (IG), le message d'état local de l'appareil (IG) étant modifié, en particulier reclassé à la hausse ou à la baisse.

8. Procédé selon la revendication de procédé qui précède immédiatement, **caractérisé en ce que** l'évaluation est effectuée de manière centralisée ou localement sur chacun des appareils médicaux (D).

9. Procédé selon l'une des revendications de procédé précédentes, l'état de l'appareil médical (D) concerné étant calculé indirectement à partir d'au moins un signal de capteur et/ou d'autres paramètres techniques de fonctionnement de l'appareil médical (D) compris dans le message d'état local (D) concerné.

10. Procédé selon l'une des revendications de procédé précédentes, **caractérisé en ce que,** pour chacun des messages d'avertissement prioritaires (pW), un mode de sortie est calculé et attribué (e), le mode de sortie comprenant la sortie d'un signal d'avertissement optique, acoustique et/ou d'un signal d'avertissement dans un autre format, de sorte que le message d'avertissement prioritaire (pW) est émis sur le dispositif de signalisation local (10) ou sur une unité de sortie dans le mode de sortie.

11. Procédé selon l'une des revendications de procédé précédentes, **caractérisé en ce qu'**une mention de blocage peut être attribuée à des messages d'état d'appareil local (IG) sélectionnés, qui indique que le message d'état d'appareil correspondant ne doit pas être classé par le message d'avertissement prioritaire (pW) calculé.

12. Procédé selon l'une des revendications de procédé précédentes, **caractérisé en ce que** la commande coordonnée modifiée n'est pas exécutée sur la base du message d'état d'appareil local (IG), mais sur la base du message d'avertissement priorisé (pW) par rapport à tous les messages d'état d'appareil.

13. Procédé selon l'une des revendications de procédé précédentes, **caractérisé en ce que** la commande coordonnée modifiée de l'appareil médical (D) peut être activée et désactivée ou est déclenchée automatiquement après la survenue d'un événement prédéfinissable.

14. Procédé selon l'une des revendications de procédé précédentes, **caractérisé en ce que** tous les messages d'alerte prioritaires calculés (pW) sont enregistrés et/ou émis de manière détaillée pour chaque appareil médical (D).

15. Procédé selon l'une des revendications de procédé précédentes, **caractérisé en ce que** le procédé comprend les étapes suivantes :
- la saisie (b1) d'une identité d'utilisateur qui est spécifique à un utilisateur de l'appareil médical (D) concerné et/ou
- la saisie (b2) d'un lieu de séjour de l'utilisateur de l'appareil médical (D) concerné,
- l'identité de l'utilisateur enregistrée et/ou le lieu de séjour enregistré étant pris en compte pour le calcul de l'alerte prioritaire (pW) pour l'appareil médical (D) concerné.

16. Procédé selon la revendication de procédé qui précède immédiatement, **caractérisé en ce que** le procédé comprend l'étape suivante, si des messages d'alerte hautement prioritaires ont été calculés pour plusieurs appareils médicaux (D) :
- génération (f) d'une commande de requête et envoi de celle-ci à des instances préconfigurées via des canaux de transmission de données préconfigurés.
